# EUROPEAN PATENT APPLICATION

(11) **EP 4 772 127 A1**
(43) Date of publication of application: **08.07.2026**
(21) Application number: 24902492.8
(22) Date of filing: 18.11.2024
(51) Int. Cl.: A61B 18/22

(54) **SEMICONDUCTOR LASER-BASED SOFT TISSUE SURGERY SYSTEM**

(30) Priority: 14.12.2023 CN 202311721575
(71) Applicant: Blueray Medical Technologies, Ltd., Xi'an, Shaanxi 710311 (CN)
(72) Inventor: MU, Liyue, Xi'an, Shaanxi 710311 (CN); ZHU, Xiang, Xi'an, Shaanxi 710311 (CN); YANG, Bing, Xi'an, Shaanxi 710311 (CN); HE, Dalin, Xi'an, Shaanxi 710311 (CN); HAN, Xiaojuan, West Xi'an, Shaanxi 710311 (CN)
(74) Representative: Viering, Jentschura & Partner mbB Patent- und Rechtsanwälte
(86) International application number: PCT/CN2024/132640
(87) International publication number: WO 2025/124070

(57) **Abstract**

The present invention provides a semiconductor laser-based soft tissue surgery system, which comprises a light source, an optical fiber output assembly, an electronic control assembly, a power assembly, and a cooling and temperature control assembly. The light source comprises a first laser device assembly, a second laser device assembly, a laser shaping assembly, and a first optical fiber coupler; the first laser device assembly is configured to emit visible light laser or emit visible light laser and near-infrared laser at the same time, and the second laser device assembly is configured to generate low-power visible light laser with the power not greater than 5 mW. The laser emitted by the first laser device assembly and the second laser device assembly is collimated and combined by means of the laser shaping assembly, coupled to the optical fiber output assembly by means of the first optical fiber coupler, and then transmitted to a target position.

## Description

### Field of the Invention

The present invention relates to a laser ablation system for soft tissue, and more particularly to a semiconductor laser-based soft tissue surgical system.

### Background

Benign Prostatic Hyperplasia (BPH) can cause urinary frequency, dysuria and incomplete bladder emptying. The surgical "gold standard" for treating BPH has been the transurethral electrosurgical resection of obstructing prostatic tissue. Since its introduction some 90 years ago, transurethral resection of the prostate (TURP) has become the most widely used surgical therapy for BPH. Unfortunately, TURP associates with numerous side effects.

In the past three decades, laser prostate surgery has become an alternative option to treat BPH, which troubles effected men with urological symptoms and decreases their quality of life. In laser prostate surgery, a high-power laser beam is delivered to target prostate tissue through an optical fiber that is introduced through an endoscope or cystoscope. The effectiveness of this treatment for BPH (or for the removal of other soft tissues) may depend on a number of factors, including wavelength, power density, illumination time and/or other factors.

Lasers are also widely used in other urological soft tissue ablations, including for the treatment of bladder tumors, treatment of ureteral strictures, laser-assisted partial nephrectomy, renal tumor interstitial laser ablation, transurethral laser urethrotomy, and in upper urinary tract tumors. Besides urological applications, lasers are also used in otolaryngology, gynecology, gastroenterology, and pulmonology for soft tissue ablations.

Conventional visible laser ablation systems implemented in laser prostate surgery may include high average power (80-180W) frequency-doubled Nd:YAG laser with a wavelength of 532nm. Since the frequency doubling is conducted by potassium titanyl phosphate (KTP) crystal or lithium borate (LBO) crystal, the green Nd:YAG laser is also called KTP laser or LBO laser. The electromagnetic radiation emitted by these systems may heat up the laser-irradiated tissue to boiling temperature, then further evaporate a top layer of the tissue and coagulate an under layer of the tissue. Green Nd:YAG laser can effectively ablate soft tissues, however, this kind of laser is inherently a complicate system, consist of high power lamp or diode laser pumping, Q-switches, sophisticated temperature controlled frequency up conversion device and other complicate subsystems, resulting high manufacture cost and low reliabilities. Besides these drawbacks, the green color laser wavelength is still considered too long to effectively vaporize soft tissues.

Holmium laser (Ho:YAG) is another kind of conventional laser system, which has a wavelength of 2140nm, much longer than the green Nd:YAG laser wavelength. By applying high power (60-120W) laser electromagnetic radiation to surgical site, laser energy produced by holmium laser system can be absorbed by water, and can thus evaporate soft tissue effectively. Due to low efficiency of soft tissue vaporization, under limited circumstances like holmium laser enucleation of the prostate (HoLEP), Ho:YAG laser surgery may produce a clinical outcome comparable with TURP. However, HoLEP technically is not easy to be mastered by general urologists, Ho:YAG laser surgery for BPH management is not yet widely accepted worldwide since its introducing to the BPH market about two decades ago.

Thulium laser is another conventional laser system commonly used for urologic tissue ablation applications. Thulium laser radiation has a wavelength of about 2000 nm, usually in continuous-wave operation mode with maximum optical power of 100-200W, produced by either solid-state Tm:YAG rod or thulium-doped fiber as lasing medium. Thulium lasers have absorption characteristics comparable with holmium lasers in water and tissue. Because of its continuous wave at infrared spectrum, the thulium laser tissue ablation is not as effective as green Nd:YAG laser is, and also can not apply to lithotripsy as holmium laser does. Because thulium laser manufacture cost is high and tissue ablation efficiency is low, this laser is not as popular as green Nd:YAG laser and holmium laser.

A various near-infrared diode lasers may be used for ablation of soft tissues in the BPH treatment. In these systems, diode lasers are operated at continuous-wave mode with wavelength in near-infrared spectrum (780-1500nm), usually at wavelength of 980nm and/or 1470nm. Electromagnetic radiations at this wavelength range could be absorbed by both water and hemoglobin in soft tissue, but absorption rates are not high resulting over 3mm penetration depth. At high power sittings (100-250W), these systems may effectively vaporize and ablate soft tissue and achieve high level of hemostasis. Compared to green Nd:YAG laser and holmium laser, these diode lasers produce too deep coagulation layer inside soft tissue, causing patients suffering several complications like obstructive necrotic tissue or bladder neck stricture. Near-infrared diode laser vaporization of the prostate, especially for 980nm laser, appears to carry a high rate of late complications, leading a repeat operation rate to more than 30%..

High power visible spectrum optical radiation has shown strong effectiveness of soft tissue ablation. In order to generate high power, the laser power density inside these conventional laser cavities is usually very high, frequently resulting optical damages on laser optics, reducing laser system operation reliability. In some cases, laser surgeries have to be stopped due to these optical damages.

For high power green Nd:YAG laser, holmium laser and thulium laser, the electric power to optical power conversion efficiency is relatively low. In some instances, this efficiency is below 3%. This may require special high power electrical outlets so these systems may operate, since any standard wall plugs (*e.g.* a 110VAC/20Amp, a 220VAC/15Amp, *etc*.) cannot provide high enough power. These conventional laser implements use solid-state materials as laser gain medium, which may dissipate huge amount of energy as heat. Thus, in order to operate these laser ablation systems normally, these surgical laser devices usually must incorporate extensive cooling systems, like high capacity liquid cooling system. These extensive cooling systems may generate loud noise inside the operation room, making physicians and nurses uncomfortable during long operation time. They also require additional power, are bulky and/or unwieldy, an/or may have other drawbacks.

Near-infrared diode lasers have overcome solid-state laser disadvantages of optical components damaging, low power conversion efficiency, and high operation noise, however, the deep coagulation layer on vaporized tissue is the major drawback, causes near-infrared diode lasers cannot be widely applied to soft tissue ablation applications.

### Summary of the Invention

One object of the present invention is to provide a semiconductor laser (diode laser) based soft tissue surgical system so as to solve the problem in the prior art that near-infrared semiconductor lasers produce a relatively deep coagulation layer during tissue vaporization.

According to one aspect of the present invention, a diode laser-based soft tissue surgical system is provided, comprising a light source, a fiber output assembly, an electronic control assembly, a power assembly, and a cooling and temperature control assembly, and may further comprise an irrigation assembly. The distinguishing features are as follows: the light source comprises a first laser assembly, a second laser assembly, a laser shaping assembly, and a first fiber coupler, and may further comprise a third laser assembly; the first laser assembly is used to emit visible laser light or a combination of visible laser light and near-infrared laser light; the second laser assembly is used to generate low-power visible laser light with a power not greater than 5 mW; the laser shaping assembly is used to collimate and combine the visible laser light and the low-power laser light, or to collimate and combine the visible laser light, near-infrared laser light, and low-power laser light; the first fiber coupler is used to couple the combined laser beam into the fiber output assembly; and the fiber output assembly is used to transmit the combined laser beam to a target location.

According to one embodiment of the present invention, the irrigation assembly is capable of generating a positive-pressure fluid. The fiber output assembly includes an optical fiber, a sleeve disposed outside the optical fiber, and a protective tube. The protective tube includes a large end, a small end, and one or more flow-through holes. The small end extends into the sleeve and is sleeved over the optical fiber. The large end is radially matched to the diameter of the sleeve and is sealingly connected thereto. A gap is formed between the sleeve and the optical fiber to serve as a fluid channel, and the light-emitting end of the optical fiber is disposed inside the protective tube. The fiber output assembly is connected to the irrigation assembly. The positive-pressure fluid generated by the irrigation assembly flows through the fluid channel and the flow-through holes to the end face of the optical fiber, thereby preventing vaporized soft tissue debris from depositing on the end face of the optical fiber.

According to one embodiment of the present invention, the end face of the optical fiber is configured as a flat end face;
The outer diameter of the protective tube is ≤ 2.5 mm;
The end face is positioned at a distance of 0.5 mm to 2.0 mm from the distal end of the protective tube.

According to one embodiment of the present invention, a reflector corresponding to the light-emitting end is disposed at the large end of the protective tube, and a light exit opening is formed in the side wall corresponding to the reflection optical path of the reflector.

According to one embodiment of the present invention, the included angle between the reflector and the central axis of the forward-emitting optical fiber is 30° to 60°.

According to one embodiment of the present invention, the end face of the optical fiber is configured as an obliquely cut or polished inclined end face, and a protective cap is sleeved over the end of the optical fiber;
The fiber transmission assembly includes a rigid water pipe and a flexible water pipe disposed outside the protective cap for delivering the positive-pressure fluid;
A fixing tube is sleeved over the rigid water pipe, the flexible water pipe, the optical fiber, and the protective cap to secure them into an integrated structure.

The rigid water pipe is disposed near the laser emission point of the side-emitting optical fiber, and the axial distance between the outlet of the rigid water pipe and the laser emission point is 1 to 2 mm;
The included angle between the inclined end face and the central axis of the optical fiber is 38 ± 1°.
According to one embodiment of the present invention, the system further includes a wavelength control module for adjusting the wavelength of the laser emitted by the light source;
The first laser assembly comprises one or more semiconductor laser modules. Each semiconductor laser module is configured to independently emit visible laser light or near-infrared laser light. A plurality of semiconductor laser modules are combined into the laser shaping assembly by fiber beam combining or spatial beam combining;
The semiconductor laser module includes one or more single-emitter semiconductor lasers, or one or more multi-emitter semiconductor lasers;
The laser shaping assembly includes one or more laser beam combiners for collimating, combining, and coupling the visible laser light and/or near-infrared laser light into the fiber output assembly, wherein the beam combining is fiber beam combining or spatial beam combining.

According to one embodiment of the present invention, the electronic control assembly includes a touch-screen display control module, a main control board, a laser power control module, a pulse control module, a first monitoring module, a second monitoring module, an Internet of Things (IoT) module, and a laser output switch;
The laser power control module is configured to control the laser output power of the light source. The pulse control module is configured to control the output laser mode, wherein the output laser modes include continuous wave mode, chopped pulse mode, and preset pulse mode;
The main control board is configured to control the operating states of the power assembly, the cooling and temperature control assembly, and the wavelength control module. The wavelength control module is configured to control the wavelength of the laser output by the light source. The first monitoring module is configured to monitor the laser power of the first laser assembly and the third laser assembly. The second monitoring module is configured to monitor the operating states of the laser shaping assembly and the first fiber coupler;
The cooling and temperature control assembly is configured to cool the light source by means of fluid-air cooling or air cooling.

According to one embodiment of the present invention, the first laser assembly is configured to emit visible laser light with a power range of 100 W to 250 W, and the wavelength of the visible laser light is 400 nm to 480 nm;
The light source further includes a third laser assembly, which is configured in the same manner as the first laser assembly and is configured to emit near-infrared laser light with a power range of 30 W to 120 W, wherein the wavelength of the near-infrared laser light is 780 nm to 1600 nm.

According to one embodiment of the present invention, the peak power of the laser emitted by the first laser assembly is 400-1000 W, the pulse width is 1-2 ms, the duty cycle is 10-20%, and the average power is 30-120 W.

Compared with the prior art, the semiconductor laser-based soft tissue surgical system provided by the present invention has the following beneficial effects:
1.The semiconductor laser modules adopted in the embodiments of the present invention can provide higher-power visible laser light and near-infrared laser light, and have a longer service life, reduced heat dissipation, and a more compact design. The visible laser light generated by the semiconductor lasers has a wavelength range of 400-700 nm, and the near-infrared laser light has a wavelength range of 780-1600 nm, which can be used respectively for ablation and coagulation of soft tissue.
2.The semiconductor laser modules adopted in the embodiments of the present invention can emit laser light in different modes, which is beneficial for efficient and high-precision ablation or coagulation of target soft tissue. Through the combination of different laser modes, the optical power density of the laser delivered to the soft tissue is increased, effectively promoting soft tissue ablation. High-peak-power pulsed visible laser light is also helpful for ablating certain urinary calculi.
3.The embodiments of the present invention adopt blue laser light with a wavelength of 400-480 nm, which, in combination with appropriate power, achieves tissue vaporization while avoiding the formation of a deep coagulation layer, thereby improving therapeutic efficacy.
4.The fiber output assembly in the embodiments of the present invention can use both forward-emitting optical fibers and side-emitting optical fibers. The light-emitting end of the optical fiber can be flexibly configured, thereby enhancing the applicability of the surgical system.

### Brief Description of the Drawings

Figure 1 is a schematic connection diagram of a semiconductor laser-based soft tissue surgical system (hereinafter referred to as the first surgical system) according to a first preferred embodiment of the present invention.
Figure 2 is a schematic connection diagram of a semiconductor laser-based soft tissue surgical system (hereinafter referred to as the second surgical system) according to a second preferred embodiment of the present invention.
Figure 3 is a schematic connection diagram of a semiconductor laser-based soft tissue surgical system (hereinafter referred to as the third surgical system) according to a third preferred embodiment of the present invention.
Figure 4 is a schematic connection diagram of a semiconductor laser-based soft tissue surgical system (hereinafter referred to as the fourth surgical system) according to a fourth preferred embodiment of the present invention.
Figure 5A illustrates one embodiment of the light source (hereinafter referred to as the first light source) of the semiconductor laser-based soft tissue surgical system described in the above four preferred embodiments of the present invention.
Figure 5B illustrates another embodiment of the light source (hereinafter referred to as the second light source) of the semiconductor laser-based soft tissue surgical system described in the above four preferred embodiments of the present invention, wherein the laser shaping assembly adopts a wavelength beam combiner.
Figure 5C illustrates another embodiment of the light source (hereinafter referred to as the third light source) of the semiconductor laser-based soft tissue surgical system described in the above four preferred embodiments of the present invention, wherein the first laser assembly and the third laser assembly are coupled to the wavelength beam combiner through free-space coupling.
Figure 6A illustrates a first semiconductor laser module, wherein the first semiconductor laser module is composed of single-emitter semiconductor lasers.
Figure 6B illustrates a second semiconductor laser module, wherein the second semiconductor laser module adopts a polarization beam combining method.
Figure 6C illustrates a third semiconductor laser module, wherein the third semiconductor laser module is composed of multi-emitter semiconductor lasers.
Figure 7A is a schematic structural diagram of a first configuration of the fiber optic output assembly according to embodiments of the present invention.
Figure 7B is a schematic structural diagram of a second configuration of the fiber optic output assembly according to embodiments of the present invention.
Figure 7C is a schematic structural diagram of a third configuration of the fiber optic output assembly according to embodiments of the present invention.
Figure 8A is a laser frequency diagram under continuous wave mode according to embodiments of the present invention.
Figure 8B is a laser frequency diagram under chopped pulse mode according to embodiments of the present invention.
Figure 8C is a laser frequency diagram under preset pulse mode according to embodiments of the present invention.
Figure 9A illustrates a liquid-air cooling and temperature control assembly according to embodiments of the present invention.
Figure 9B illustrates an air cooling and temperature control assembly according to embodiments of the present invention.

### Detailed Description

The following description is provided to disclose the present invention so as to enable those skilled in the art to implement the invention. The preferred embodiments described below are provided only as examples, and other obvious modifications may occur to those skilled in the art. The basic principles of the invention defined in the following description may be applied to other embodiments, modifications, improvements, equivalent solutions, and other technical solutions without departing from the spirit and scope of the present invention.

Those skilled in the art should understand that, in the disclosure of the present invention, terms such as "longitudinal," "transverse," "upper," "lower," "front," "rear," "left," "right," "vertical," "horizontal," "top," "bottom," "inner," and "outer" indicate orientations or positional relationships based on those shown in the drawings. These terms are used only for convenience in describing the invention and for simplifying the description, and do not indicate or imply that the referenced device or element must have a particular orientation or must be constructed and operated in a particular orientation. Therefore, these terms should not be construed as limiting the present invention.

It should be understood that the term "a" or "one" means "at least one" or "one or more." That is, in one embodiment, the quantity of an element may be one, while in another embodiment, the quantity of that element may be multiple. The term "a" or "one" should not be construed as limiting the quantity.

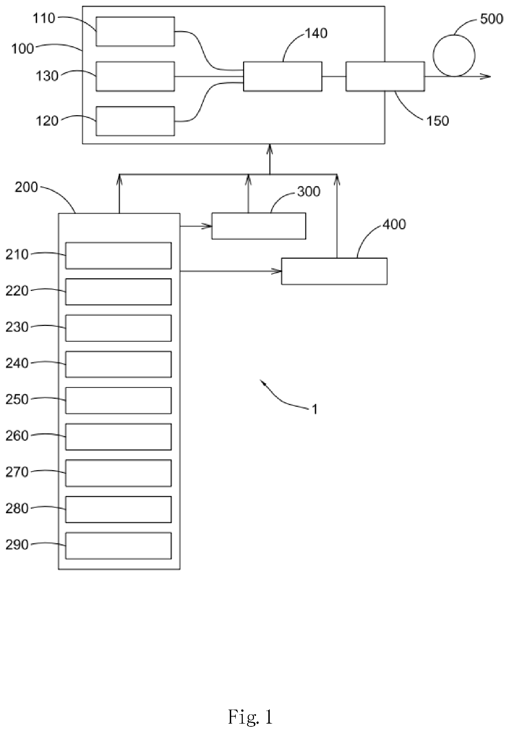

Figure 1 illustrates a semiconductor laser-based soft tissue surgical system according to a first preferred embodiment of the present invention, hereinafter referred to as the first surgical system 1. The first surgical system **1** may be specifically configured for ablation of prostate tissue or other soft tissue. The first surgical system **1** includes a light source **100,** a fiber output assembly **500,** an electronic control assembly **200,** a power assembly **300,** and a cooling and temperature control assembly **400.**

The light source **100** is configured to generate laser light of a predetermined wavelength and predetermined output power, and includes a first laser assembly **110,** a third laser assembly **130,** a second laser assembly **120,** a laser shaping assembly **140,** and a first fiber coupler **150.**

The first laser assembly **110** is a fiber-coupled and/or free-space-coupled laser device, which can generate visible laser light of predetermined wavelength and power, such as blue light at 450 ± 20 nm, and/or green light at 520 ± 20 nm, and/or violet light at 405 ± 5 nm. The predetermined visible light power is sufficiently high to meet the requirements of soft tissue ablation and coagulation.

The third laser assembly **130** is configured to emit laser light at near-infrared wavelengths, such as 980 ± 20 nm, 808 ± 20 nm, 1470 ± 20 nm, and/or 1550 ± 20 nm. For laser prostatectomy, the average output power of the first laser assembly **110** is preferably in the range of 100 W to 250 W, and the average output power of the third laser assembly **130** is preferably in the range of 30 W to 120 W.

The second laser assembly **120** is configured to generate visible laser light of a wavelength different from that of the first laser assembly **110,** and can generate low-power laser light not exceeding 5 mW, serving as an aiming beam for the first surgical system 1 to indicate the predetermined target position where the first laser assembly **110** and the third laser assembly **130** emit laser light.

The laser shaping assembly **140** is configured to combine the laser beams emitted by the first laser assembly **110,** the third laser assembly **130,** and the second laser assembly **120** into a single collimated beam, and transmit the beam to the first fiber coupler **150,** which ultimately couples the beam into the fiber output assembly **500.** The laser emitted from the fiber output assembly **500** may have one or more wavelengths.

The fiber output assembly **500** includes an optical fiber **510,** which is used to transmit laser energy to the patient's prostate tissue or other target soft tissue to ablate or coagulate the tissue. The end face of the optical fiber **510** may be a flat end face **511** or an inclined end face.

The electronic control assembly **200** is configured to control the operation of the first surgical system **1.** It includes a display control module **270,** a main control board **210,** a laser power control module **220,** a pulse control module **230,** a first monitoring module **240,** a second monitoring module **250,** an Internet of Things (IoT) module **280,** a wavelength control module **290,** and a laser output switch **260.**

The display control module **270** is configured to provide all necessary interfaces between the operator and the first surgical system **1.** It includes one or more setting pages that allow the operator to set and read operating parameters and feedback of the first surgical system **1.** The display control module **270** may also allow authorized service engineers to perform authorized software and firmware system upgrades, and to read, retrieve, and transmit the operational history data of the soft tissue resection system via wired or wireless means. In the case of wireless communication, the IoT module **280** provides the communication protocol.

The main control board **210** may include one processor or multiple processors. The processor(s) may be centralized on the main control board **210** or distributed among other modules of the electronic control assembly **200** to control corresponding modules. In this embodiment, the electronic control assembly **200** may further include processors integrated with other components of the first surgical system **1,** as well as a host system or central processing unit configured to control and/or read data from the first surgical system **1.**

The main control board **210** includes at least one embedded system, one or more laser pump power supply circuits, one or more communication circuits, one or more flash memory circuits, one or more analog-to-digital (A/D) acquisition circuits, one or more signal modulation circuits, one or more cooling device drivers, and one or more wavelength control module drivers.

The central processing unit (CPU) includes one or more 32-bit high-performance microcontrollers, such as the STM32 F4 series based on ARM Cortex-M4, or other types of high-performance microcontrollers capable of supporting operating frequencies above 100 MHz. The main control board **210** may be implemented in hardware, software, firmware, or a combination thereof.

The laser power control module **220** is configured to control the output power of the first laser assembly **110** and the third laser assembly **130** and to receive their power feedback. For example, the laser power control module **220** may include one or more drivers that communicate with the first laser assembly **110** and the third laser assembly **130.**

The pulse control module **230** may include a power control board and a laser pulse modulation driver, connected to one or more DC power supplies and to the main control board **210** containing one or more processors. The pulse control module **230** can implement continuous wave mode, chopped pulse mode, preset pulse mode, or other output modes by performing pulse modulation on the first laser assembly **110** and the third laser assembly **130.**

The first monitoring module **240** is configured to monitor in real time the laser power status of the first laser assembly **110** and the third laser assembly **130** and to feed the laser power status back to the laser power control module **220.** The laser power feedback signals of the first laser assembly **110** and the third laser assembly **130** originate from electrical signals generated by laser power sensors located inside the laser assemblies. The laser power sensors may be thermopile laser sensors, photodiode laser sensors, and/or other types of laser sensors.

The second monitoring module **250** receives signals from laser power detection devices disposed in the laser shaping assembly **140,** signals from temperature sensors, and fiber coupling status signals from the output of the first fiber coupler **150,** and transmits these signals to the laser power control module **220.**

The laser output switch **260,** preferably a foot switch, is configured to turn the laser output of the light source **100** on or off.

The IoT module **280** may include an embedded subsystem board supporting wired and wireless communication with external networks, such as Ethernet, Wi-Fi networks, Bluetooth communication, and 2G/3G/4G/5G wireless networks. The IoT module **280** is configured to provide communication between the first surgical system **1** and a central computer or other IoT systems, and may be implemented in hardware, software, firmware, or a combination thereof.

The wavelength control module **290** is configured to adjust the laser wavelength of the light source **100** and is controlled by the main control board **210** to preset the laser status of the first laser assembly **110** and/or the third laser assembly **130.** After a predetermined laser wavelength is selected by the wavelength control module **290,** the laser is output through the fiber output assembly **500.**

Furthermore, each of the display control module **270,** the main control board **210,** the laser power control module **220,** the pulse control module **230,** the first monitoring module **240,** and the second monitoring module **250** may be implemented in hardware, software, firmware, or a combination thereof. The modules may be executed locally, or one or more modules may be executed remotely in conjunction with one or more other modules.

The power assembly **300** includes an isolation transformer required by regulatory standards, a power switch, one or more power protection circuits, and one or more AC-to-DC power converters. The power assembly **300** may be controlled by the main control board **210** to supply power to the light source **100.** It may also receive input power from an external power source, and part of the input power may be used to drive the light source **100** and/or other components of the first surgical system **1.**

The cooling and temperature control assembly **400** is controlled by the main control board **210** and is configured to cool the first laser assembly **110,** the third laser assembly **130,** the laser shaping assembly **140,** the first fiber coupler **150,** and/or other components of the first surgical system **1.** The cooling and temperature control assembly **400** may receive information from temperature sensors disposed within the first laser assembly **110,** the third laser assembly **130,** the laser shaping assembly **140,** the first fiber coupler **150,** and/or other components of the first surgical system **1,** and cool the light source **100** in real time based on this information. The cooling and temperature control assembly **400** may dissipate heat through a heat sink or a fluid-air heat exchanger.

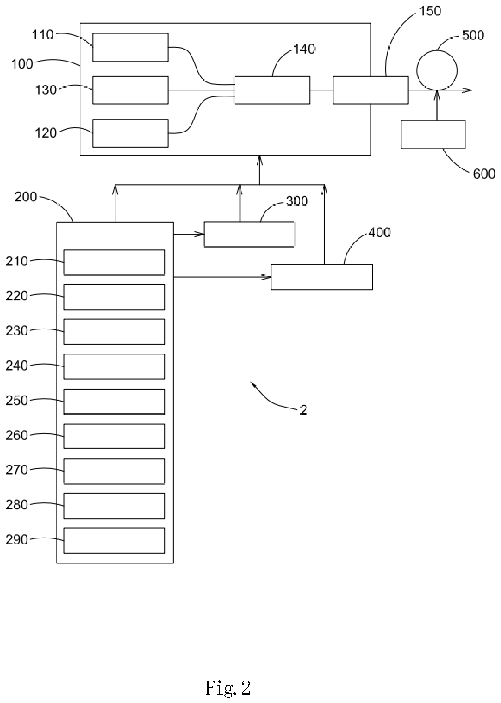

Figure 2 illustrates a semiconductor laser-based soft tissue surgical system according to a second preferred embodiment of the present invention, hereinafter referred to as the second surgical system **2.** The difference from the first surgical system **1** is that the second surgical system **2** further includes an irrigation assembly **600.** The irrigation assembly **600** may be integrated into the second surgical system **2** or may be independently applied during surgery. The irrigation assembly **600** may be an active fluid pump, preferably a peristaltic pump, powered by the power assembly **300** or independently powered by a pump controller. The fluid in the irrigation assembly **600** flows into the fiber output assembly **500** to prevent tissue debris from accumulating on the fiber output assembly **500** during surgery.

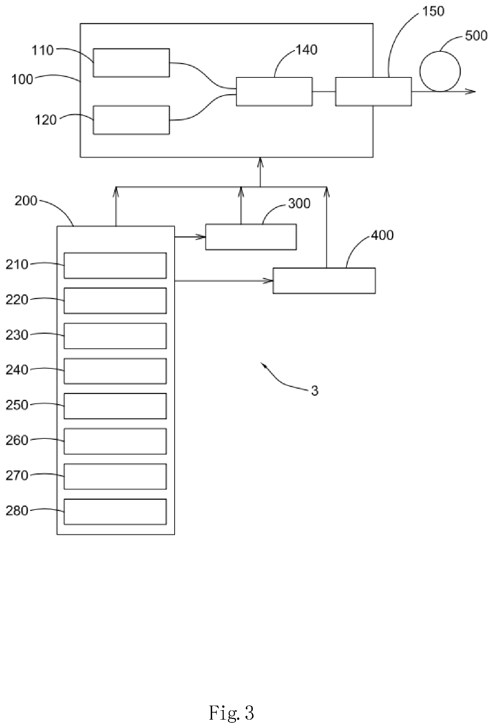

Figure 3 illustrates a semiconductor laser-based soft tissue surgical system according to a third preferred embodiment of the present invention, hereinafter referred to as the third surgical system **3.** Compared with the first surgical system **1,** the third surgical system **3** omits the third laser assembly **130** and the wavelength control module **290.**

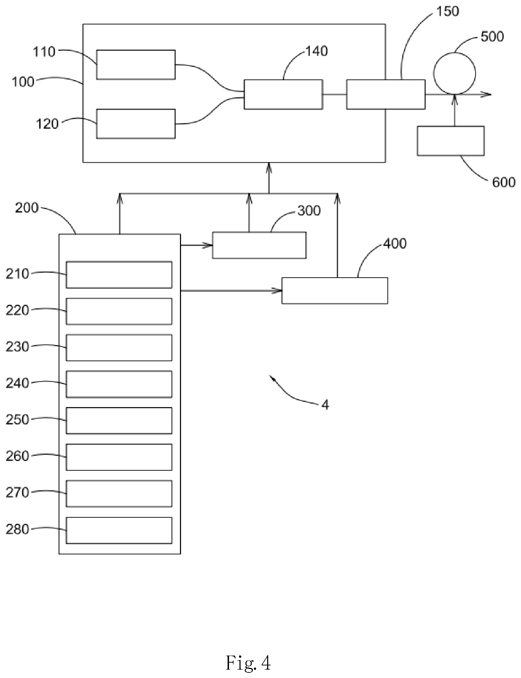

Figure 4 illustrates a semiconductor laser-based soft tissue surgical system according to a fourth preferred embodiment of the present invention, hereinafter referred to as the fourth surgical system **4.** Compared with the second surgical system **2,** the fourth surgical system **4** omits the third laser assembly **130** and the wavelength control module **290.**

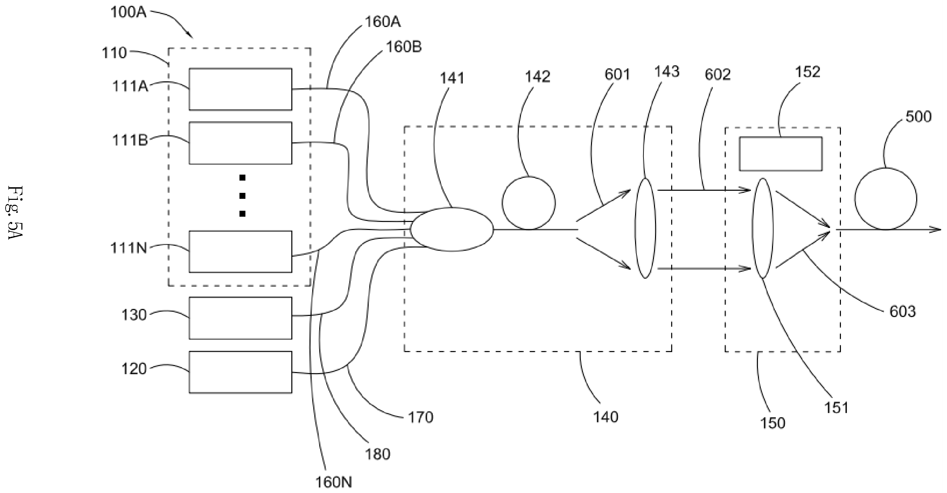

The first light source **100A** shown in Figure 5A is one embodiment of the light source **100.** The first light source **100A** is configured to generate laser light of predetermined wavelength and predetermined output power, and includes the first laser assembly **110,** the third laser assembly **130,** the second laser assembly **120,** the laser shaping assembly **140,** and the first fiber coupler **150.**

The first laser assembly **110** includes N semiconductor laser modules **111,** where N is an integer equal to or greater than 1, denoted as **111A** to **111N.** The N semiconductor laser modules **111** are respectively input to the laser shaping assembly **140** through corresponding N first output fibers **160,** denoted as **160A to 160N.**

The semiconductor laser modules **111** may be configured to emit one or more visible laser wavelengths or a combination of visible and near-infrared laser wavelengths, which are adjusted by the wavelength control module **290.** The third laser assembly **130** is configured in the same manner as the first laser assembly **110** and outputs near-infrared laser light. The output end of the third laser assembly **130** is coupled to a third output fiber **180.** The second laser assembly **120** is coupled to a second output fiber **170.**

The laser shaping assembly **140** includes a fiber beam combiner **141** and a first collimator **143.** The fiber beam combiner **141** is disposed at the output ends of the first output fibers **160,** the second output fiber **170,** and the third output fiber **180.** The first output fibers **160,** the second output fiber **170,** and the third output fiber **180** are fusion-spliced together using a fusion splicer and connected to the input end of the fiber beam combiner **141.** The output end of the fiber beam combiner **141** is provided with a fourth output fiber **142.** Therefore, the fiber beam combiner **141** includes multiple input fibers and one fourth output fiber **142.**

Provided that the product of the numerical aperture (NA) of the input fibers and the sum of the core areas of each input fiber is less than the product of the NA and core area of the output fiber, multiple input laser beams can be effectively combined into a single output laser beam.

The input end of the first collimator **143** is connected to the output end of the fourth output fiber **142.** A first output beam **601** from the fourth output fiber **142** is divergent and is collimated by the first collimator **143.** The first collimator **143** consists of one or more spherical or aspherical lenses. The lens surfaces are coated with anti-reflection coatings to reduce reflection of laser light from the first laser assembly **110** and/or the third laser assembly **130.**

After collimation by the first collimator **143,** the first output beam **601** becomes a second output beam **602,** which is output through the first fiber coupler **150.**

The first fiber coupler **150** includes a coupling lens **151** and a coupling status monitor **152.** The coupling lens **151** may consist of one or more spherical or aspherical lenses. To reduce optical energy loss, the coupling lens **151** is coated with an anti-reflection coating corresponding to the second output beam **602.** The third output beam **603** from the coupling lens **151** is focused into the fiber output assembly **500.**

The coupling status monitor **152** includes a laser power detection sensor and a temperature sensor for monitoring the power of the third output beam **603** and the temperature of the coupling lens **151,** and transmitting the information to the second monitoring module **250.**

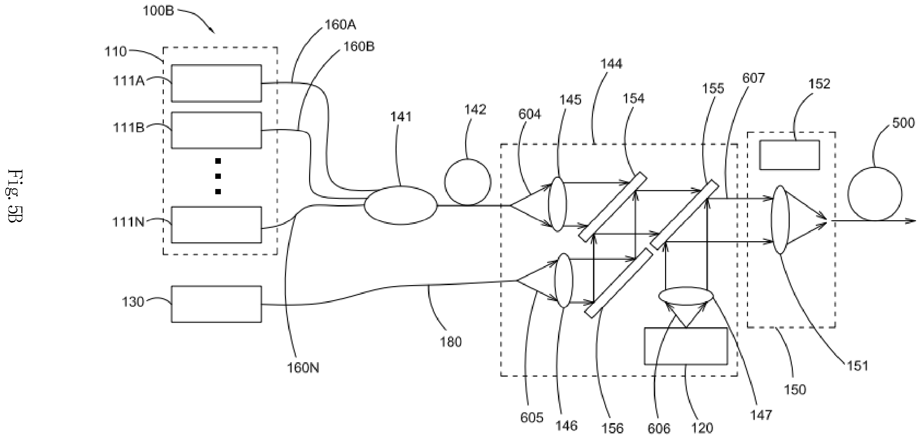

The second light source **100B** shown in Figure 5B is another embodiment of the light source **100.** Compared with the first light source **100A,** the second light source **100B** further includes a wavelength beam combiner **144.**

The laser outputs from the first laser assembly **110,** the third laser assembly **130,** and the second laser assembly **120** are coupled to the laser shaping assembly **140** and then transmitted to the first fiber coupler **150.** The fiber beam combiner **141** is configured to combine the corresponding N first output fibers **160** of the first laser assembly **110** and output them to the fourth output fiber **142,** wherein N is an integer equal to or greater than 1. The third laser assembly **130** is coupled to the third output fiber **180.**

The fourth output fiber **142** and the third output fiber **180** output divergent laser beams to the wavelength beam combiner **144.** The corresponding divergent beams are denoted as a fourth output beam **604** and a fifth output beam **605,** respectively. The divergence angles of the beams are determined by the numerical apertures (NA) of the fourth output fiber **142** and the third output fiber **180.**

The wavelength beam combiner **144** includes:
- A second collimator **145** and a third collimator **146** disposed respectively at the output ends of the fourth output fiber **142** and the third output fiber **180;**
- A first wavelength combining mirror **154** and a second wavelength combining mirror **155** disposed sequentially at the output end of the second collimator **145;**
- A first reflector **156** corresponding to the third collimator **146;** and
- A fourth collimator **147** corresponding to the output end of the second laser assembly **120.**

The second collimator **145** and the third collimator **146** are configured to collimate the fourth output beam **604** and the fifth output beam **605,** respectively. Each of the second collimator **145** and the third collimator **146** includes one or more spherical or aspherical lenses, coated with anti-reflection coatings corresponding to the fourth output beam **604** and the fifth output beam **605,** respectively.

The collimated fifth output beam **605** is reflected by the first reflector **156** to the reflective surface of the first wavelength combining mirror **154.** The collimated fourth output beam **604** passes through the first wavelength combining mirror **154** and reaches the second wavelength combining mirror **155.**

The first wavelength combining mirror **154** is a planar optical substrate coated on both sides, with a 45° anti-reflection/transmission coating for the collimated fourth output beam **604** and a 45° high-reflection coating for the collimated fifth output beam **605.** The first wavelength combining mirror **154** combines the collimated fourth output beam **604** and the fifth output beam **605.**

The second laser assembly **120** emits a sixth output beam **606,** which is collimated by the fourth collimator **147** and then directed to the second wavelength combining mirror **155.** The second wavelength combining mirror **155** combines the sixth output beam **606** with the fourth output beam **604** and the fifth output beam **605** into a seventh output beam **607.** The seventh output beam **607** is transmitted to the first fiber coupler **150** and then focused into the fiber output assembly **500.**

In other embodiments of the present invention, the second laser assembly **120** may also be delivered to the first fiber coupler **150** through an optical fiber.

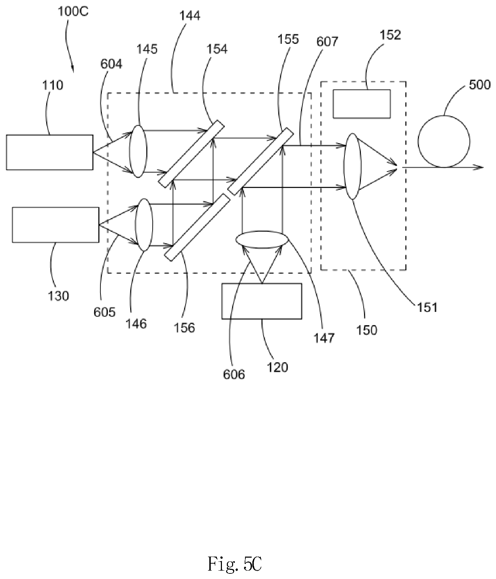

The third light source **100C** shown in Figure 5C is another embodiment of the light source **100.** Unlike the second light source **100B,** in the third light source **100C,** the first laser assembly **110** and the third laser assembly **130** are coupled to the wavelength beam combiner **144** through free-space coupling. Correspondingly, the second collimator **145** and the third collimator **146** are respectively provided for the first laser assembly **110** and the third laser assembly **130.**

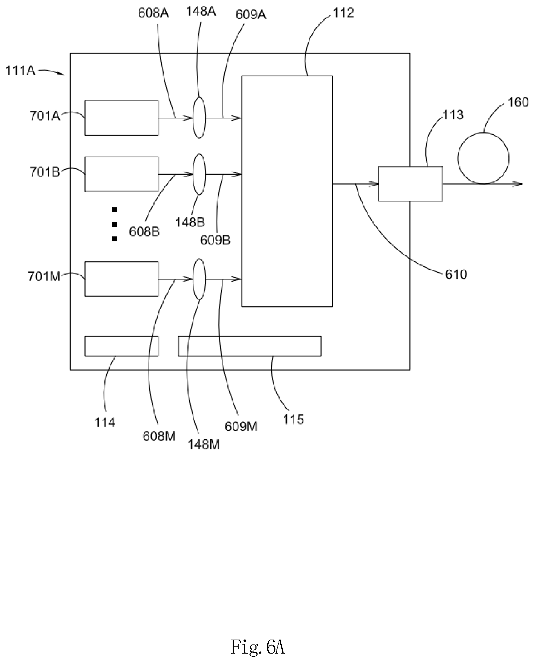

The first semiconductor laser module **111A** shown in Figure 6A is one embodiment of the semiconductor laser module **111.** It includes M single-emitter semiconductor lasers **701,** denoted as **701A** to **701M.** The single-emitter semiconductor lasers **701A** to **701M** are configured to emit visible laser light, such as 450 ± 20 nm, 520 ± 20 nm, or 405 ± 5 nm, and/or near-infrared light, such as 980 ± 20 nm, 808 ± 20 nm, 1470 ± 20 nm, or 1550 ± 20 nm.

The first semiconductor laser module **111A** uses a spatial beam combining method to combine the beams emitted by the single-emitter semiconductor lasers **701A to 701M** and couple them into the first output fiber **160.**

The single-emitter semiconductor lasers **701** emit eighth output beams **608,** denoted as **608A to 608M.** The beams **608A** to **608M** are respectively collimated by corresponding fifth collimators 148 **(148A** to **148M)** to obtain ninth output beams **609,** denoted as **609A** to **609M.**

The fifth collimator **148** may consist of a pair of fast-axis collimators (FAC) and slow-axis collimators (SAC), or may consist of one or more spherical or aspherical lenses. The surface of the fifth collimator **148** is coated with an anti-reflection coating matching the laser wavelength emitted by the corresponding single-emitter semiconductor laser **701.**

The ninth output beams **609A to 609M** are directed to a first beam shaper **112.** The first beam shaper **112** is a focusing or directional optical component, which may consist of a pair of spherical or aspherical cylindrical lenses, or two or more reflectors and/or other optical components.

After shaping, a tenth output beam **610** is produced. The tenth output beam **610** is focused and coupled into the first output fiber **160** through a second fiber coupler **113.**

In order to obtain high-brightness or high power-density laser output at the fourth output fiber **142** of the fiber beam combiner **141,** the core diameter of the first output fiber **160** needs to be between 50 µm and 600 µm, and the numerical aperture (NA) needs to be between 0.1 and 0.25.

In the first semiconductor laser module **111A,** the single-emitter semiconductor lasers **701,** the first beam shaper **112,** and the second fiber coupler **113** all generate heat and thus act as heat sources. Therefore, a cooling device **114** is provided to cool these components. The cooling device **114** may be a liquid cooling and/or air cooling assembly configured to conduct and dissipate heat from these hot spots to one or more externally disposed heat exchangers.

In other embodiments of the present invention, the cooling device **114** may also be an air-cooling device, assembled together with the semiconductor laser module **111** as a single unit, including one or more thermoelectric (TE or TEC) cooling devices and fans.

The first semiconductor laser module **111A** further includes one or more operational status monitors **115,** which may include temperature sensors, photodiode laser sensors, and/or fiber sensors, configured to provide real-time signals such as temperature and output power of the first semiconductor laser module **111A** to corresponding modules of the electronic control assembly **200.**

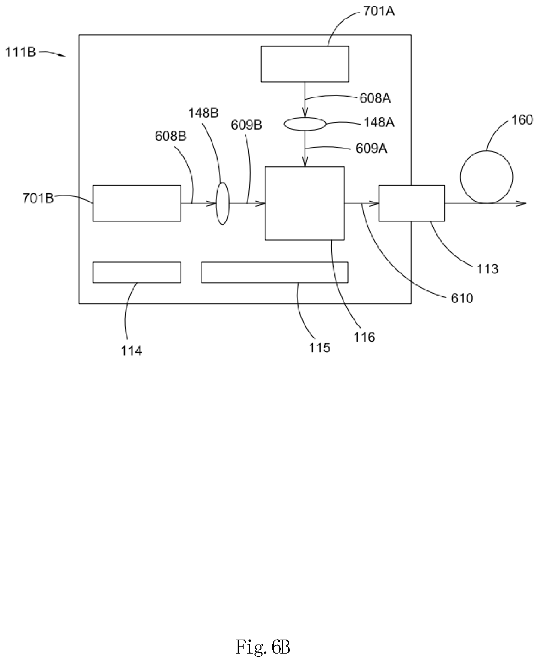

The second semiconductor laser module **111B** shown in Figure 6B is another embodiment of the semiconductor laser module **111.** It adopts a polarization beam combining method and includes two single-emitter semiconductor lasers **701A** and **701B.** Their arrangement causes the polarization directions of the eighth output beams **608A** and **608B** to be perpendicular to each other.

The eighth output beams **608A** and **608B** are collimated by corresponding fifth collimators **148A** and **148B** to obtain ninth output beams **609A** and **609B,** respectively. The ninth output beams **609A** and **609B** are combined by a polarization beam combiner **116** to form the tenth output beam **610.** The tenth output beam **610** is then focused and coupled into the first output fiber **160** through the second fiber coupler **113.**

To obtain higher laser output power, the single-emitter semiconductor lasers **701** may be replaced with multi-emitter semiconductor lasers **702,** and other corresponding optical components, such as the fifth collimator **148,** may be adaptively modified accordingly.

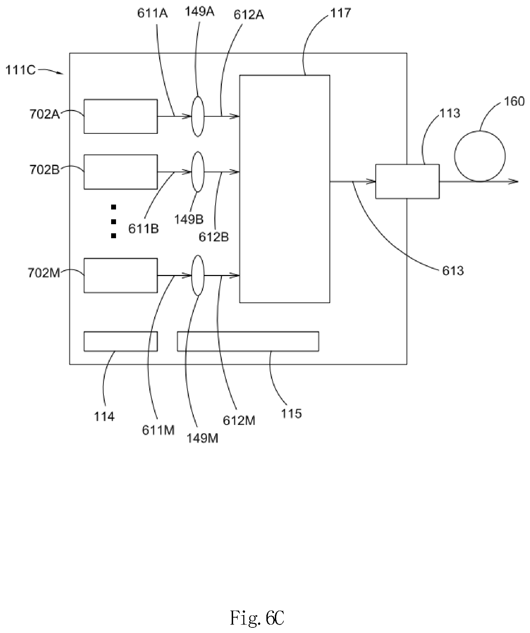

The third semiconductor laser module **111C** shown in Figure 6C is another embodiment of the semiconductor laser module **111.** The difference from the first semiconductor laser module **111A** is that multi-emitter semiconductor lasers **702** are used in place of single-emitter semiconductor lasers **701.**

M multi-emitter semiconductor lasers **702,** denoted as **702A** to **702M,** are configured to emit visible laser light, such as 450 ± 20 nm, 520 ± 20 nm, or 405 ± 5 nm, or near-infrared light, such as 980 ± 20 nm, 808 ± 20 nm, 1470 ± 20 nm, or 1550 ± 20 nm.

The eleventh output beams **611A** to **611M** emitted by the multi-emitter semiconductor lasers **702A** to **702M** are respectively collimated by corresponding sixth collimators **149A** to **149M** to produce twelfth output beams **612A** to **612M.**

The sixth collimator **149** may consist of a pair of fast-axis collimators (FAC) and slow-axis collimators (SAC), or a combined FAC-SAC collimator. The surface of the sixth collimator **149** is coated with an anti-reflection coating matching the laser wavelength emitted by the corresponding multi-emitter semiconductor laser **702.**

The twelfth output beams **612** are guided to a second beam shaper **117** to produce a thirteenth output beam **613.** The second beam shaper **117** is a focusing or directional optical component. The thirteenth output beam **613** is focused and coupled into the first output fiber **160** through the second fiber coupler **113.**

In order to maintain high laser brightness or high laser power density at the fiber output assembly **500,** the core diameter of the first output fiber **160** needs to be between 100 µm and 600 µm, and the numerical aperture (NA) needs to be between 0.1 and 0.25.

In other embodiments of the present invention, the semiconductor laser module **111** may be composed of multiple single-emitter semiconductor lasers **701,** or multiple multi-emitter semiconductor lasers **702,** or a combination of multiple single-emitter semiconductor lasers **701** and multi-emitter semiconductor lasers **702.** The module may emit visible laser light and/or near-infrared light at any wavelength within the range of 400 nm to 1600 nm. As long as the laser power and wavelength meet the requirements for soft tissue ablation, the soft tissue can be ablated.

Since the high-power visible laser emitted from the fiber output assembly **500** can effectively vaporize the target soft tissue and generate a large amount of soft tissue debris, some of the debris may adhere to the light-emitting end face of the optical fiber **510** within the fiber output assembly **500.** This may cause laser energy accumulation at the fiber output assembly **500,** resulting in heating or even melting of the optical fiber **510.** Therefore, it is necessary to prevent tissue debris from adhering to the light-emitting end face of the fiber assembly **500.**

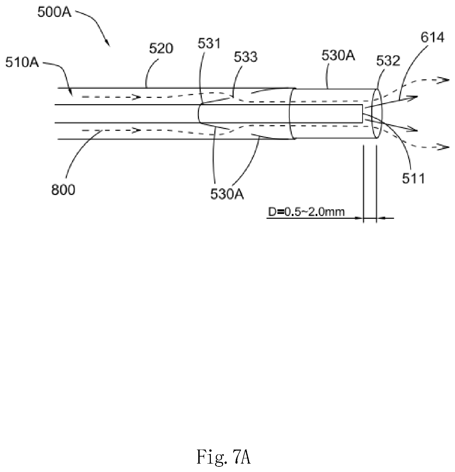

The first fiber output assembly **500A** shown in Figure 7A is one embodiment of the fiber output assembly **500.** It includes a first optical fiber **510A,** a sleeve **520** disposed outside the first optical fiber **510A,** and a first protective tube **530A** located at the light-emitting end of the optical fiber.

The first optical fiber **510A** is one embodiment of the optical fiber **510** and includes a flat end face **511** perpendicular to the axis of the first optical fiber **510A.** The flat end face **511** could be perpendicular to the axis of the optical fiber **510** and is formed by cutting or polishing. A first emitted laser beam **614,** composed of visible light and/or near-infrared light, is emitted from the flat end face **511.**

The protective tube **530A** is one embodiment of the protective tube **530** and is preferably made of medical-grade stainless steel. Its cross-section is preferably circular and includes a small end **531,** a large end **532,** and one or more flow-through holes **533.**

The small end **531** and the large end **532** are coaxial with the first optical fiber **510A.** The inner diameter of the small end **531** is slightly larger than the outer diameter of the first optical fiber **510A,** allowing the first optical fiber **510A** to pass through. The outer side of the large end **532** is sealingly connected to the end of the sleeve **520.** In order for the fiber output assembly **500** to pass through the working channel of a surgical endoscope, the outer diameter of the large end **532** is not greater than 2.5 mm.

The small end **531** may be fastened to the first optical fiber **510A** or bonded thereto using epoxy resin. The small end **531** may be mechanically fixed or chemically bonded with epoxy resin, with mechanical fixation without epoxy being preferred. The shrinkage rate of epoxy resin may change the local refractive index of the first optical fiber **510A,** thereby damaging the fiber. If epoxy resin must be used, ultraviolet curing may be applied to achieve a low linear shrinkage rate. The epoxy resin may be transparent to allow visible laser radiation to pass through, or may be colored, preferably white, to reflect visible laser light back into the first optical fiber **510A.**

To better protect the light-emitting end face from soft tissue debris, the flat end face **511** is positioned inside the first protective tube **530A** and is spaced 0.5 mm to 2.0 mm from the distal end of the large end **532** of the first protective tube **530A.** This prevents the flat end face **511** from contacting the target soft tissue during laser operation.

A gap is formed between the sleeve **520** and the first optical fiber **510A** to serve as a fluid channel. One or more flow-through holes **533** are formed in the first protective tube **530A** near the small end **531.** Positive-pressure fluid **800** flows through the fluid channel and the flow-through holes **533** into the interior of the large end **532.**

The distal end of the fluid channel is connected to an active fluid pump or a fluid bag. The active fluid pump is preferably a peristaltic pump. The fluid bag is preferably a fluid reservoir or storage bag positioned at a higher elevation. During surgery, the positive-pressure fluid **800** may be the same fluid used in the surgical procedure, preferably saline solution, and has a pressure higher than that of the surrounding surgical environment.

At the light-emitting end of the first optical fiber **510A,** the positive-pressure fluid **800** forms a jet surrounding the flat end face **511,** thereby preventing soft tissue debris from adhering and keeping the flat end face **511** clean.

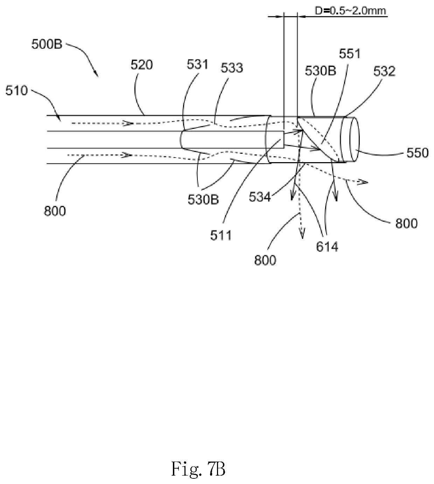

For certain surgical applications, such as vaporization of prostate tissue, side emission of the laser facilitates easier surgical operation. The second fiber output assembly **500B** shown in Figure 7B is another embodiment of the fiber output assembly **500.** Compared with the first fiber output assembly **500A,** the second fiber output assembly **500B** further includes a second reflector **550** and a second protective tube **530B.**

The second protective tube **530B** is another embodiment of the protective tube **530.** Unlike the first protective tube **530A,** it further includes a light exit opening **534.**

The flat end face **511** of the second fiber assembly **500B** may be perpendicular to the axis of the optical fiber **510** or disposed at a certain angle. The second reflector **550** is cylindrical and is disposed inside the large end **532** of the second protective tube **530B.** One end of the second reflector **550** is provided with a beveled reflective surface **551** corresponding to the flat end face **511.** The reflective surface **551** reflects the first emitted laser beam **614** to form side-emitting light.

The light exit opening **534** provided on the second protective tube **530B** is sufficiently large to allow the reflected first emitted laser beam **614** to pass through without obstruction.

The positive-pressure fluid **800** flows through the flow-through holes **533** from the outer side of the small end **531** into the inner side of the large end **532** and then exits through the light exit opening **534.**

The second reflector **550** may be made of optical materials such as fused quartz or metals such as aluminum. The angle between the reflective surface **551** and the central axis of the second reflector **550** may be 38°, 45°, or any angle between 30° and 60°, producing corresponding reflection angles of 52°, 45°, or between 60° and 30°, respectively. The reflective surface **551** may be optically polished and coated with a high-reflection coating for all wavelengths of the first emitted laser beam **614.**

The second reflector **550** is fixed to the large end **532** by epoxy bonding or mechanical fastening. If epoxy bonding is used, the epoxy resin may be cured by ultraviolet light, heat curing, or roomtemperature curing.

The sleeve **520** is connected to the second protective tube **530B** but does not cover the light exit opening **534.** The positive-pressure fluid **800** forms a positive-pressure jet at the light exit opening **534** to prevent soft tissue debris from depositing on the reflective surface **551** and the flat end face **511.**

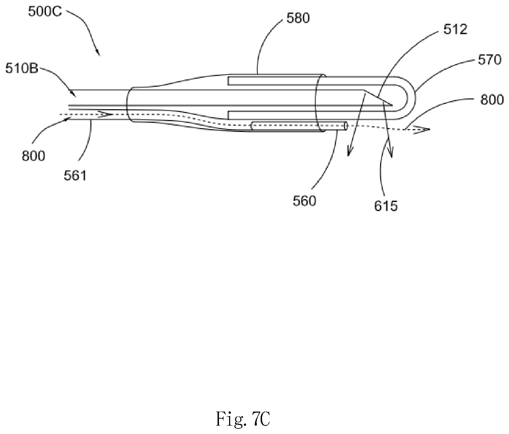

The third fiber assembly **500C** shown in Figure 7C is another embodiment of the fiber output assembly **500.** The third fiber assembly **500C** includes a second optical fiber **510B,** a flexible water tube **561,** a rigid water tube **560,** a protective cap **570,** and a fixing tube **580.**

The second optical fiber **510B** is another embodiment of the optical fiber **510** and has an obliquely cut or polished inclined end face **512,** which enables internal total reflection of a second emitted laser beam **615** composed of visible light and/or near-infrared light. For improved total internal reflection, the angle between the inclined end face **512** and the axis of the second optical fiber **510B** is 38 ± 1°.

The protective cap **570** is preferably made of quartz or fused quartz, with a length of 12 ± 5 mm, an outer diameter of 1.5-2.2 mm, and an inner diameter larger than the outer diameter of the second optical fiber **510B.** One end of the protective cap **570** is closed and the other end is open. The second optical fiber **510B** is inserted into the protective cap **570,** with the inclined end face **512** positioned near the closed end.

Adhesive epoxy resin may be applied at the junction between the open end of the protective cap **570** and the second optical fiber **510B.** To prevent potential damage to the second optical fiber **510B** caused by visible laser leakage around the epoxy region, the epoxy resin is preferably ultraviolet-cured with a low linear shrinkage rate, preferably less than 0.08%, to prevent changes in the refractive index of the second optical fiber **510B** after UV curing.

The flexible water tube **561** is preferably a heat-shrink tube, with one end connected to the positive-pressure fluid **800** and the other end connected to the rigid water tube **560.** The rigid water tube **560** is disposed on the side of the protective cap **570** and is preferably made of medical-grade stainless steel, with a smaller diameter and thinner wall thickness than the protective cap **570.**

The fixing tube **580** has a thin-walled structure and is transparent or white in color to prevent damage from high-power visible laser light. The fixing tube **580** secures the protective cap **570,** the flexible water tube **561,** the rigid water tube **560,** and the second optical fiber **510B** together into an integrated structure. The fixing tube **580** is preferably a heat-shrink tube.

The outlet of the rigid water tube **560** is positioned 1-2 mm away from the laser emission point. At the outlet of the rigid water tube **560,** the positive-pressure fluid **800** forms a jet that flows across the laser emission point, preventing soft tissue debris deposition and keeping the protective cap **570** clean.

The electronic control assembly **200** and its submodule, the pulse control module **230,** may modulate the first laser assembly **110** and the third laser assembly **130** in real time in continuous wave mode, chopped pulse mode, preset pulse mode, or other electronically controlled modes.

Through the display control module **270,** the operator or surgeon may set the laser emission mode of the first surgical system **1** or the second surgical system **2.**

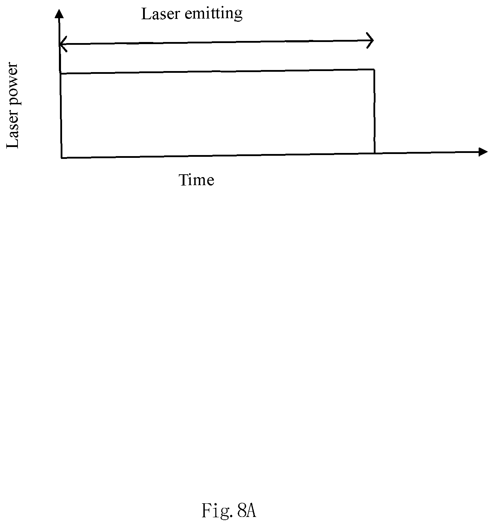

Figure 8A shows a surgical laser emission frequency diagram in continuous wave (CW) mode. Generally, the continuous wave mode has relatively high average laser power, enabling effective soft tissue vaporization, soft tissue removal, and hemostatic procedures. Lower laser power in CW mode is beneficial for soft tissue coagulation.

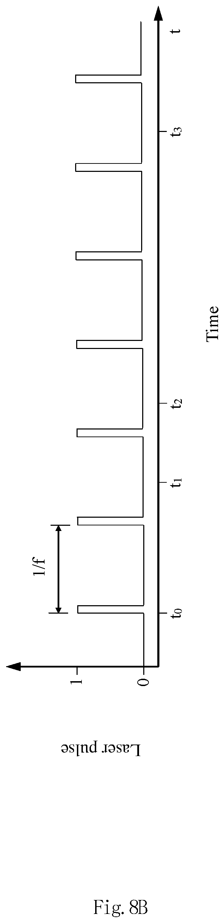

Figure 8B shows a surgical laser emission frequency diagram in chopped pulse mode. A chopped pulse mode or pulse mode with high peak power and low average power allows surgeons to achieve precise tissue resection and tissue coagulation.

Under the conditions of a visible surgical laser system with pulse peak power of 400-1000 W, average power of 30-120 W, pulse width of 1-2 ms, and duty cycle of 10-20%, the laser pulse energy of the first surgical system **1** and the second surgical system **2** may reach 0.4 J to 2.0 J, which is sufficient to ablate common urinary calculi.

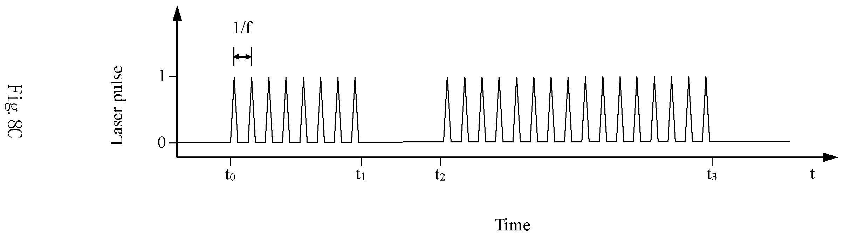

In addition to the chopped pulse mode, the pulse control module **230** may also generate any form of preset pulse, as shown in Figure 8C.

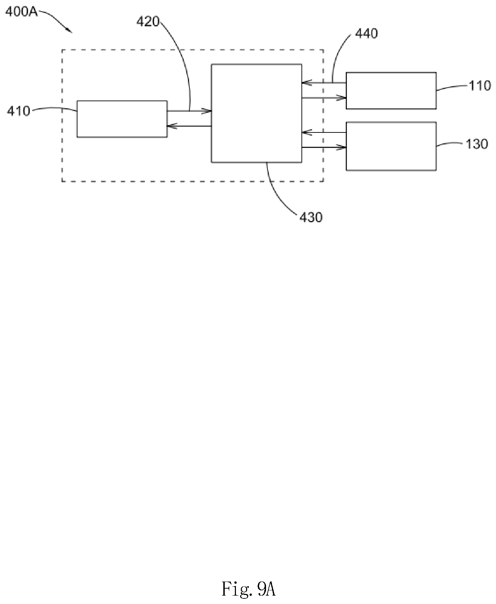

The first cooling and temperature control assembly **400A** shown in Figure 9A is one embodiment of the cooling and temperature control assembly **400.** The first cooling and temperature control assembly **400A** reduces the heat generated by the first laser assembly **110** and the third laser assembly **130** through a fluid-air heat exchanger **410.** The preferred fluid is water. The fluid-air heat exchanger **410** may consist of metal tubes, metal housings, and/or metal plates, as well as one or more fans.

The fluid-air heat exchanger **410** is connected through a first pipeline **420** to one or more liquid containers. A water pump assembly **430** is disposed in the liquid container. The water pump assembly **430** delivers circulating cooling water to the first laser assembly **110** and the third laser assembly **130** through a second pipeline **440.**

The water pump assembly **430** may include one or more liquid pumps. The liquid container is provided with temperature sensors, pressure sensors, and liquid level sensors.

In other embodiments, the first cooling and temperature control assembly **400A** may not be connected to temperature sensors and may not perform temperature regulation.

In further embodiments, the first cooling and temperature control assembly **400A** may be a temperature-regulated liquid chiller that operates in real time based on feedback from temperature sensors. It may use a refrigeration compressor and include a condenser, refrigerant such as R134a, a liquid pump, a reservoir, one or more refrigerant-air heat exchangers, and/or one or more thermoelectric cooling devices and other components.

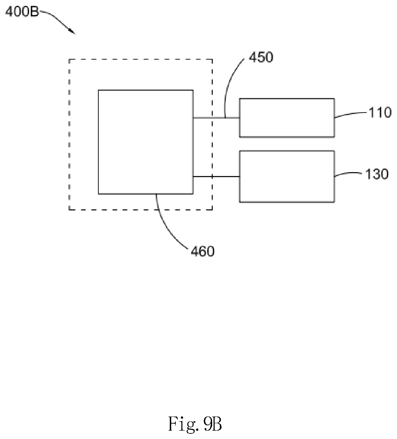

The second cooling and temperature control assembly **400B** shown in Figure 9B is another embodiment of the cooling and temperature control assembly **400.** In this embodiment, a heat conduction device **450** connects the heat source to a heatsink **460,** and heat is dissipated through thermal conduction.

In the above embodiments, the first laser assembly **110,** the third laser assembly **130,** the laser shaping assembly **140,** the first fiber coupler **150,** and other components or modules all generate heat. The fiber beam combiner **141** in the laser shaping assembly **140** or the second fiber coupler **113** may also serve as heat sources.

The heat conduction device **450** may be a heat pipe, vapor chamber, heat plate, or other type of heat conduction component. The heatsink **460** includes one or more thermoelectric cooling (TEC) temperature regulation devices and fans.

One or more heat sources connected to the heat conduction device **450** may not require temperature regulation. The heatsink **460** may be a conventional heat sink with or without fans for heat dissipation.

The working principles of the above preferred embodiments of the present invention are as follows:
The visible laser employed is a blue laser with a wavelength of 400-480 nm. The energy of blue photons is as high as 2.59-3.11 eV. Blood and soft tissue in the human body can effectively absorb blue photons. The optical absorption coefficient of hemoglobin at 450 nm blue light and 532 nm green light is comparable (approximately 200/cm), which means that both 450 nm blue light and 532 nm green light can effectively ablate soft tissue containing blood.

For soft tissue with low blood content, such as coagulated tissue, according to the light absorption theory of biological organic molecules, such soft tissue generally has a higher optical absorption coefficient for blue light than for green light. Therefore, at the same optical power density, blue laser achieves faster tissue ablation than green laser.

An in vitro experimental report shows that in terms of tissue removal rate (in mm³/s), under identical tissue conditions, a 120 W blue laser vaporizes human prostate tissue at twice the rate of a 532 nm LBO green laser of the same power.

To obtain higher output power, the semiconductor laser module **111** may include multiple single-emitter semiconductor lasers **701,** which achieve high-power output through spatial arrangement, beam shaping, and coupling. The semiconductor laser module **111** may also include multiple multi-emitter semiconductor laser modules **702.**

In fiber coupling of multiple single-emitter semiconductor lasers **701,** the single-emitter semiconductor lasers **701** are arranged spatially in one or more rows. Collimating lenses are placed in front of each emitter to collimate the laser beam along both the fast axis and the slow axis. For multi-emitter laser bar configurations with fiber coupling, fast-axis collimators (FAC) and slow-axis collimators (SAC) are used to collimate the beams.

These collimated beams are further converged by optical concentrators and then focused into an optical fiber through focusing lenses. To maintain reasonably high laser brightness, the core diameter of the optical fiber is less than 600 µm.

For laser prostatectomy, the average output power of the laser is preferably in the range of 100 W to 300 W. If a single semiconductor laser module **111** cannot reach this optimal power range, multiple semiconductor laser modules **111** may be used to increase the laser output power.

A fiber beam combining and shaping device, which may be a fiber beam combiner or a spatial beam combiner, can assist in beam combining. The fiber beam combining and shaping device has multiple inputs and one output. For a fiber beam combiner, when the product of the input fiber NA and the sum of the core areas of each input fiber is less than the product of the output fiber NA and its core area, multiple input laser beams can be combined into a single output laser beam. The beam combining efficiency is high, typically above 85%. Although a fiber beam combiner does not increase laser brightness, it can significantly increase optical power.

When a single semiconductor laser module **111** is capable of generating more than 100 W of visible light power, only one semiconductor laser module **111** may be required. The semiconductor laser module **111** may be fiber-coupled or free-space-coupled. The final laser output may be emitted directly or coupled through the optical fiber **510** and then delivered to the target tissue for ablation and/or coagulation. The optical fiber **510** may be a disposable surgical fiber.

As is well known, in laser surgery, lasers that have a high absorption rate in tissue, hemoglobin, or water can produce a thinner coagulation layer. A thinner coagulation layer enables faster postoperative recovery for patients but is less convenient for achieving hemostasis. However, bleeding is unavoidable during laser prostatectomy. Therefore, to provide surgeons with an effective tool for hemostasis, it is necessary to use a semiconductor laser capable of generating high-power near-infrared laser light.

The present invention may also provide only near-infrared laser light. The near-infrared laser light is generated by near-infrared semiconductor lasers with wavelengths between 780 nm and 1600 nm. In particular, commercially available near-infrared semiconductor lasers at 810 ± 20 nm, 980 ± 20 nm, and 1470 ± 20 nm are optimal choices.

High-power fiber-coupled or free-space-coupled near-infrared semiconductor lasers may be combined through a fiber beam combiner and shaping device and ultimately delivered to the target soft tissue via a disposable surgical fiber.

For better and more effective laser coagulation, the near-infrared laser may generate optical power of 30-120 W in continuous wave mode or pulse mode, which is critical for hemostasis.

The surgical system of the present invention may simultaneously provide visible laser light and near-infrared laser light. Multiple visible single-emitter semiconductor lasers and multiple near-infrared single-emitter semiconductor lasers may be spatially arranged in one or more rows, with beam shaping and convergence, and then focused into a single optical fiber, or collimated in free space.

Such a single semiconductor laser module **111** may achieve an average optical power exceeding 100 W in the visible spectrum and up to 120 W in the near-infrared spectrum. The final laser output may be delivered directly or coupled again through an optical fiber and transmitted to the target soft tissue via a disposable surgical optical fiber for ablation and/or coagulation.

Efficient laser soft tissue ablation generates a large amount of debris, some of which may deposit on the light-emitting end face of the optical fiber, resulting in laser absorption and even fiber damage.

Blue photons have higher photon energy than photons of longer wavelengths. Compared with longer-wavelength light, soft tissue debris absorbs blue light more strongly. For lasers with lower photon energy, such as high-power green lasers and high-power infrared lasers, the heat generated by debris absorption at the fiber output end is insufficient to cause rapid melting of the fiber tip. However, this is not the case for blue light.

Some experiments have shown that when the blue laser power exceeds 80 W, the heat generated by debris absorbing blue light at the fiber output end may cause catastrophic melting of the fiber tip.

To prevent tissue debris from depositing on the fiber output end face, especially in high-power blue laser soft tissue ablation, the fiber tip must be provided with a self-cleaning mechanism.

In the present invention, positive-pressure fluid **800** is used to form a jet that flows across the light-emitting end of the high-power visible laser fiber to prevent debris deposition. The fluid may be the same as the fluid used in surgery, preferably saline.

To form a fluid jet that adequately covers the laser fiber emission point, the jet fluid pressure must be higher than the surrounding environmental pressure, and the fluid must maintain a continuous jet in the front the laser fiber emission point.

To generate the desired jet fluid pressure, an irrigation assembly **600** may be used. The irrigation assembly **600** may include an active fluid pump or one or more passive fluid bags suspended at a relatively higher position.

When the fiber output assembly **500** adopts the form of the first fiber output assembly **500A,** the end face of the optical fiber is centered within the protective tube, which is preferably made of stainless steel. The high-pressure jet fluid within the protective tube can flow across the end face of the optical fiber. For better protection, the laser emission point of the optical fiber end face may be positioned approximately 0.5 to 2 mm behind the outlet of the protective tube.

When the fiber output assembly **500** adopts the form of the second fiber output assembly **500B,** the second reflector directs the laser beam to a desired angle to form side emission. The angle between the reflector and the central axis of the corresponding optical fiber is 30° to 60°, and the reflector is coated with a high-reflection coating for the output laser. The end face of the optical fiber and the reflective surface of the second reflector are both surrounded by high-pressure jet fluid.

When the fiber output assembly **500** adopts the form of the third fiber output assembly **500C,** the rigid water tube **560** is made of stainless steel and is installed directly below the protective cap **570** at the light-emitting end of the side-emitting fiber, approximately 1 to 2 mm behind the laser emission point. The fluid jet flowing through the stainless steel tube prevents any debris from depositing on the protective cap **570.**

The semiconductor laser module **111** that emits visible light may operate in continuous wave mode or pulse mode. In continuous wave mode, it can provide relatively high average power, mainly for rapid soft tissue ablation. In pulse mode, it provides higher peak power and lower average power, which is advantageous for precise soft tissue ablation and fragmentation of urinary calculi.

When the semiconductor laser module **111** emitting visible light is powered by one or more pulsed power supplies, the electrically pumped pulsed current may reach 1.5 to 10 times its maximum continuous-mode current, thereby generating visible laser pulses with peak power several times higher than the continuous wave power. The laser pulse width, pulse repetition rate, and duty cycle may be adjusted by modifying the characteristics of the pump current pulses.

By controlling the visible laser pulse energy, the surgeon can precisely remove soft tissue as needed. Because visible light, especially blue wavelengths, is strongly absorbed by many solid materials, including urinary calculi, high-peak-power blue laser pulses are capable of fragmenting urinary stones.

With a peak power of 400-1000 W and a pulse width of 1-2 ms, the laser pulse energy may reach 0.4-2.0 J, which is sufficient to break common urinary calculi. The duty cycle may be 10-20%, and the average power may be within the range of 30-120 W.

The semiconductor laser module **111** emitting visible light may further improve electro-optical conversion efficiency. In some embodiments, a GaN blue semiconductor laser module **111** typically has a DC electro-optical conversion efficiency exceeding 25%. When output from the fiber assembly, the overall electro-optical conversion efficiency may reach 15% or more. Even when powered by an AC power source, the final electro-optical conversion efficiency may exceed 10%.

One advantage of high electro-optical conversion efficiency is that it facilitates implementation of the soft tissue resection system in various treatment environments, especially in environments where high-power electrical sources are not readily available (such as hospital wards, physicians' offices, or patients' homes).

The single-emitter semiconductor laser **701** may be a GaN blue semiconductor laser module packaged in TO9 or TO56 form. TO-packaged GaN blue semiconductor lasers can operate at ambient housing temperatures up to 65°C.

The GaN-based visible-light-emitting semiconductor laser module **111,** benefiting from high power conversion efficiency, low heat dissipation, and high allowable operating temperature, allows a non-temperature-regulated water-air heat exchanger to dissipate heat into the air. Although a liquid chiller may be used to stabilize the coolant temperature, multiple non-temperature-regulated water-air cooling configurations can significantly reduce the noise of the surgical system, thereby providing a quieter environment in the operating room.

The semiconductor laser module **111** may be cooled and temperature-regulated by one or more thermoelectric (TE or TEC) cooling devices or by a liquid chiller. On one hand, all-solid-state air-cooled TEC devices can eliminate any liquid-related reliability issues in the surgical laser system. On the other hand, a liquid chiller can make the physical size of the surgical laser system more compact.

In some embodiments, the surgical system includes a semiconductor laser module **111** packaged within a fiber-coupled assembly, a free-space-coupled assembly, or a combination of fiber coupling and free-space coupling assemblies, as well as a fiber output assembly **500,** a power assembly **300,** a cooling assembly, and an electronic control assembly **200.** The laser may be output at a predetermined wavelength and predetermined output power.

The fiber output assembly **500** delivers the laser to the patient's soft tissue to ablate the target tissue. The semiconductor laser module **111** may emit visible laser light, such as violet laser light at 405 ± 5 nm, green laser light at 520 ± 20 nm, or laser light of other visible colors. The fiber output assembly **500** includes an optical fiber.

In some cases, the end face of the optical fiber includes a forward-emitting end or a side-emitting end. These emitting ends are provided with an active cleaning assembly that allows positive-pressure fluid **800** to flush away tissue debris during laser ablation.

The power assembly **300** may receive input power from an external source and supply part or all of the power to the surgical system. The cooling assembly may cool various heat sources during operation. The electronic control assembly **200** may control the operating states of various components and parts of the surgical system.

The semiconductor laser module **111** may be various types of high-power fiber-coupled or free-space-coupled semiconductor lasers. The single-emitter semiconductor laser **701** may have different packaging forms (for example, TO package, COS package, C-mount package, etc.), and the multi-emitter semiconductor laser **702** may be packaged in a laser bar configuration.

The semiconductor laser module **111** may further include a power supply assembly, a control assembly, a wavelength selection control assembly, and a cooling assembly. The laser generation assembly may generate laser light of one or more predetermined wavelengths. The power supply assembly may supply power to the laser generation assembly, and the electronic control assembly **200** ensures that the laser is output from the source at a predetermined output power.

The wavelength control module **290** may operate together with the laser generation assembly and the power assembly **300** to generate laser wavelengths required for tissue ablation and/or tissue coagulation. In some cases, the output wavelength for tissue ablation and that for tissue coagulation may be different. The cooling assembly ensures stable and reliable laser output.

As another embodiment employing a high-power fiber-coupled and/or free-space-coupled semiconductor laser module **111** emitting visible light at power levels exceeding 100 W, the blue semiconductor laser module **111** does not dissipate as much energy in the form of heat as conventional visible laser systems with similar output power.

Other visible laser systems used for soft tissue ablation generally employ solid-state crystals as the laser gain medium (such as neodymium-doped yttrium aluminum garnet (Nd:YAG)). The heat generated as a byproduct of electro-optical conversion requires extensive cooling systems to ensure that the laser system is not damaged by excessive heat.

In contrast, a high-power fiber-coupled and/or free-space-coupled visible semiconductor laser module **111,** particularly a fiber-coupled blue semiconductor laser module **111,** may be maintained at a safe operating temperature by a cooling assembly using a liquid cooling device with relatively low cooling capacity, such as multiple non-temperature-regulated water-air heat exchangers, a small temperature-regulated chiller, or multiple thermoelectric (TEC) temperature control devices.

This reduces the noise of the surgical system and optimizes its overall size and weight, thereby making the system more convenient to use.

In the present invention, the first laser assembly **110** is composed of one or more fiber-coupled and/or free-space-coupled semiconductor laser modules **111.** The wavelength emitted from the semiconductor laser module **111** may be a visible wavelength, or a combination of visible and near-infrared wavelengths.

To obtain higher optical output power, each fiber-coupled and/or free-space-coupled semiconductor laser module **111** may further include two or more single-emitter semiconductor lasers **701** and/or one or more multi-emitter semiconductor lasers **702** with predetermined wavelengths. Through spatial multiplexing, polarization beam combining, and/or wavelength multiplexing, the laser beams are coupled into a single optical fiber with a core diameter smaller than 600 µm, or directly injected into a disposable surgical optical fiber with a core diameter smaller than 1000 µm.

In some embodiments, the first laser assembly **110** may include one or more fiber-coupled and/or free-space-coupled semiconductor laser modules **111** and one or more pulse control modules **230.**

The first laser assembly **110** may generate high-power laser light at one or more predetermined wavelengths by combining fiber-coupled semiconductor laser modules into a single output.

The pulse control module **230** adjusts the pump current in pulse mode or other modes to modulate the laser output with temporal characteristics, thereby providing laser pulses at a predetermined frequency and predetermined pulse width.

The electronic control assembly **200** may control the peak power and average power of the laser. By controlling the visible laser pulse energy, the surgeon can precisely ablate soft tissue as needed.

When the visible laser pulse peak power is greater than 400 W, the pulse width is less than 5 ms, and the duty cycle is less than 20%, the surgical laser system can fragment common urinary calculi.

In some embodiments of the present invention, laser generation from the visible semiconductor laser module **111** does not require bulk optical components. Conventional solid-state laser systems require highly reflective bulk optical components to form a resonant cavity, such as high-reflectivity optical elements for forming a laser cavity and laser wavelength conversion crystals such as potassium titanyl phosphate (KTP) or lithium triborate (LBO).

The absence of such bulk optical components in the visible semiconductor laser module **111** of the present invention greatly improves the reliability of the surgical system in the operating room.

Those skilled in the art should understand that the above description and the embodiments shown in the accompanying drawings are provided by way of example only and do not limit the present invention. The objectives of the present invention have been fully and effectively achieved. The functional and structural principles of the present invention have been illustrated and described in the embodiments. Without departing from these principles, the embodiments of the present invention may be subject to various variations or modifications.

## Claims

1. A semiconductor-laser-based soft tissue surgical system, **characterized in that** it comprises a light source (100), a fiber output assembly (500), an electronic control assembly (200), a power supply assembly (300), and a cooling and temperature control assembly (400);
the light source (100) is configured to emit visible laser light, or a combination of visible laser light and near-infrared laser light;
the fiber output assembly (500) is configured to transmit the laser emitted by the light source (100) to a target site;
the electronic control assembly (200) is configured to control the light source (100), the power supply assembly (300), and the cooling and temperature control assembly (400);
the power supply assembly (300) is configured to supply power to the light source (100), the electronic control assembly (200), and the cooling and temperature control assembly (400);
the cooling and temperature control assembly (400) is configured to dissipate heat from the light source (100) by means of fluid-to-air cooling or air cooling.

2. The semiconductor-laser-based soft tissue surgical system according to claim 1, further comprising an irrigation assembly (600);
the irrigation assembly (600) is connected to the fiber output assembly (500) and is configured to generate a positive-pressure fluid.

3. The semiconductor-laser-based soft tissue surgical system according to either claim 1 or claim 2, wherein the light source (100) comprises a first laser assembly (110), a second laser assembly (120), a laser shaping assembly (140), and a first fiber coupler (150);
the first laser assembly (110) is configured to emit visible laser light, or a combination of visible laser light and near-infrared laser light;
the second laser assembly (120) is configured to emit visible laser light, with a laser power not greater than 5 mW;
the laser shaping assembly (140) is configured to collimate and combine the laser beams emitted by the first laser assembly (110) and the second laser assembly (120);
the first fiber coupler (150) is configured to couple the laser emitted from the laser shaping assembly (140) into the fiber output assembly (500).

4. The semiconductor-laser-based soft tissue surgical system according to claim 3, wherein the first laser assembly (110) comprises one or more semiconductor laser modules (111), and a plurality of semiconductor laser modules (111) are combined by fiber beam combining or free-space beam combining;
the semiconductor laser modules (111) are configured to emit visible laser light, or a combination of visible laser light and near-infrared laser light.

5. The semiconductor-laser-based soft tissue surgical system according to claim 4, wherein the semiconductor laser module (111) comprises a single-emitter semiconductor laser (701) and/or a multi-emitter semiconductor laser (702);
the single-emitter semiconductor laser (701) and the multi-emitter semiconductor laser (702) are configured to emit visible laser light, or a combination of visible laser light and near-infrared laser light.

6. The semiconductor-laser-based soft tissue surgical system according to any one of claims 3 to 5, wherein the light source (100) may further comprise a third laser assembly (130);
the third laser assembly (130) is arranged in the same manner as the first laser assembly (110) and is configured to emit near-infrared laser light;
the laser shaping assembly (140) is further configured to collimate and combine the laser beams emitted by the first laser assembly (110), the second laser assembly (120), and the third laser assembly (130), wherein the beam combining method may be fiber beam combining or free-space beam combining.

7. The semiconductor-laser-based soft tissue surgical system according to either claim 1 or claim 2, wherein the fiber output assembly (500) comprises an optical fiber (510);
the optical fiber (510) is configured to transmit the laser emitted by the light source (100), and an end face of the optical fiber (510) may be a flat end face (511) perpendicular to the central axis, or an angled end face (512) forming an angle of 38 ± 1° with respect to the central axis.

8. The semiconductor-laser-based soft tissue surgical system according to claim 7, wherein the fiber output assembly (500) may further comprise a sleeve (520) and a protective tube (530);
the sleeve (520) is disposed on the outer side of the optical fiber (510), and a gap is provided between the sleeve (520) and the optical fiber (510);
the end face of the optical fiber (510) is disposed within the protective tube (530).

9. The semiconductor-laser-based soft tissue surgical system according to claim 8, wherein the protective tube (530) comprises a small end (531), a large end (532), and a flow-through hole (533);
the small end (531) extends into the sleeve (520) and is sleeved onto the optical fiber (510);
the large end (532) is radially matched to the diameter of the sleeve (520) and is sealingly connected to the sleeve (520);
the flow-through hole (533) is located between the large end (532) and the small end (531), and is disposed within the sleeve (520).

10. The semiconductor-laser-based soft tissue surgical system according to claim 9, wherein an outer diameter of the large end (532) is ≤ 2.5 mm;
a distance between the end face of the optical fiber (510) and the large end (532) is 0.5 mm to 2.0 mm.

11. The semiconductor-laser-based soft tissue surgical system according to claim 9, wherein the fiber output assembly (500) may further comprise a second reflector (550);
the reflector (550) forms an angle of 30° to 60° with respect to the central axis of the optical fiber (510), and is disposed within the protective tube (530) for reflecting the laser transmitted by the optical fiber (510);
the protective tube (530) may further comprise a light-emitting aperture (534);
the light-emitting aperture (534) is located outside the sleeve (520) and is positioned opposite to the second reflector (550).

12. The semiconductor-laser-based soft tissue surgical system according to claim 7, wherein the fiber output assembly (500) may further comprise a protective cap (570), a flexible water tube (561), a rigid water tube (560), and a fixing tube (580);
the protective cap (570) is made of glass or quartz material, is fixed to the optical fiber (510), and is configured to protect the optical fiber (510);
the rigid water tube (560) is closely attached to a side surface of the protective cap (570) and is configured to transmit a positive-pressure fluid and flush the protective cap (570), wherein an axial distance between an outlet of the rigid water tube (560) and a laser-emitting position of the optical fiber (510) is 1 mm to 2 mm;
the flexible water tube (561) is sealingly connected to the rigid water tube (560) and is configured to transmit the positive-pressure fluid;
the fixing tube (580) is configured to fix the optical fiber (510), the protective cap (570), the rigid water tube (560), and the flexible water tube (571).

13. The semiconductor-laser-based soft tissue surgical system according to either claim 1 or claim 2, wherein the electronic control assembly (200) comprises a display control module (270), a main control board (210), a laser power control module (220), a pulse control module (230), a first monitoring module (240), a second monitoring module (250), an Internet of Things (IoT) module (280), and a laser output switch (260);
the display control module (270) is configured to display and control system status parameters; the laser power control module (220) is configured to control the output laser power of the light source (100);
the pulse control module (230) is configured to control the laser output mode of the light source (100), wherein the output laser modes include a continuous wave mode, a chopped pulse mode, and a preset pulse mode;
the first monitoring module (240) is configured to monitor the laser power of the first laser assembly (110) and the third laser assembly (130);
the second monitoring module (250) is configured to monitor the operating status of the laser shaping assembly (140) and the first fiber coupler (150);
the IoT module (280) is configured to provide communication between the system and a central computer or other Internet of Things systems;
the laser output switch (260) is configured to control turning on and off of the light source (100); the main control board (210) is configured to control the operating status of the power supply assembly (300) and the cooling and temperature control assembly (400).

14. The semiconductor-laser-based soft tissue surgical system according to claim 13, wherein the electronic control assembly (200) may further comprise a wavelength control module (290);
the main control board (210) may further be configured to control an operating state of the wavelength control module (290);
the wavelength control module (290) is configured to adjust a wavelength of the laser emitted by the light source (100).

15. The semiconductor-laser-based soft tissue surgical system according to any one of claims 1 to 14, wherein the light source (100) emits visible laser light having a wavelength of 400-480 nm and a power range of 100 W to 250 W.

16. The semiconductor-laser-based soft tissue surgical system according to any one of claims 1 to 14, wherein the light source (100) is further configured to emit near-infrared laser light having a wavelength of 780-1600 nm and a power range of 30 W to 120 W.

17. The semiconductor-laser-based soft tissue surgical system according to any one of claims 1 to 14, wherein the light source (100) is further configured to emit pulsed laser light having a peak power of 400-1000 W, a pulse width of 1-2 ms, a duty cycle of 10-20%, and an average power of 30-120 W.
